# EUROPÄISCHE PATENTSCHRIFT

(11) **EP 3 410 955 B1**
(45) Veröffentlichungstag und Bekanntmachung des Hinweises auf die Patenterteilung: **18.03.2020**
(21) Anmeldenummer: 17708165.0
(22) Anmeldetag: 06.02.2017
(51) Int. Cl.: A61B 17/06

(54) **MEDIZINISCHES GERÄT**
MEDICAL DEVICE
APPAREIL MÉDICAL

(30) Priorität: 04.02.2016 DE 102016101937
(43) Veröffentlichungstag der Anmeldung: 12.12.2018
(73) Patentinhaber: DOCI, Violeta, 22111 Hamburg (DE); DOCI, Donata Michelle, 20099 HAMBURG (DE)
(72) Erfinder: DOCI, Violeta, 22099 Hamburg (DE)
(74) Vertreter: Sonn & Partner Patentanwälte
(86) Internationale Anmeldenummer: PCT/EP2017/052570
(87) Internationale Veröffentlichungsnummer: WO 2017/134311

(56) Entgegenhaltungen:
- US-A- 5 562 685
- US-A1- 2009 082 788
- US-A1- 2012 071 719

## Beschreibung

### Gebiet der Erfindung

Die vorliegende Erfindung betrifft ein medizinisches Gerät, mit dem Fäden in Gewebe eingebracht werden können. In diesem Sinne handelt es sich also vor allem um ein chirurgisches Gerät. Das Gerät kann im heilend-medizinischen Bereich, aber auch in kommerziellen Bereichen Anwendung finden.

### Hintergrund der Erfindung

Die internationale Patentanmeldung WO 01/30245 A1 offenbart ein chirurgisches Instrument, mit dem chirurgische Nähte gelegt werden können. Dieses Instrument besteht im Wesentlichen aus zwei röhrenförmigen Elementen, wobei die erste Röhre eine zentrale Achse bildet und die zweite Röhre als Helix um diese Achse herumgeführt wird. Das Ende der zweiten Röhre ist zugespitzt. Das Instrument soll im Wesentlichen dazu dienen, eine chirurgische Naht zu legen, bei der der Faden spiralförmig geführt wird. Dazu wird die helix-förmige Röhre entlang der vorgesehenen Fadenbahn durch Gewebe geführt. Der Faden kann dabei in der Vorschubphase oder in der Entnahmephase im Gewebe platziert werden.

Die US-Patentanmeldung US 2012/0136200 offenbart ein anderes chirurgisches Instrument. Dieses ist speziell für chirurgische Eingriffe am Herzen ausgelegt. Das Gerät weist ein Griffstück auf, an dessen vorderen Ende mittig eine Punktiernadel gehalten wird. Um die Punktiernadel herum ist eine helix-förmige Nadel angeordnet. Die Punktiernadel kann in eine Herzwand eingefügt werden. Um die durch die Punktiernadel festgelegte Stelle kann mit Hilfe der helix-förmigen Nadel ein Faden platziert werden.

Die beiden in diesen Patentanmeldungen offenbarten Instrumente sind für spezielle Formen der Platzierung chirurgischer Fäden gestaltet. Die chirurgischen Fäden sollen aber im Wesentlichen in klassischer Weise dem Legen einer chirurgischen Naht dienen, also der zumindest temporären Verbindung von Gewebe. Beide Instrumente lassen sich nur anwenden, wenn gleichzeitig mindestens zwei Nadeln eingesetzt werden und dementsprechend auch zwei Nadeln Gewebe durchtrennen.

Die US 5,562,685 A offenbart ein Operationswerkzeug mit einer spiralförmigen Nadel, hinter deren Spitze eine Durchgangsöffnung zur Aufnahme eines Fadens angeordnet ist. Im hinteren Bereich ist ein Griff angeordnet.

Die US 2009/0082788 A1 offenbart ein gebogenes Operationsinstrument, an dessen vorderen Ende eine Art Zange ausgebildet ist, die beispielsweise mit Hilfe von Scherenschenkeln betätigt werden kann. In der Nähe des Gelenkpunktes der Zange befindet sich eine Öffnung. Um die Zange zu betätigen, befindet sich im Inneren des Operationsinstruments ein Aktuator in Form eines Fadens oder Drahtes.

Die US 2012/0071719 A1 offenbart ein medizinisches Gerät mit zwei spiralförmigen Körpern mit jeweils einem abstehenden Anker, wobei in dem Anker Öffnungen vorgesehen sind.

Die vorliegende Erfindung möchte ein neuartiges medizinisches Gerät zur Verfügung stellen. Dieses soll zum schonenden und kontrollierenden Legen chirurgischer Fäden dienen, welche nicht unbedingt der Verbindung zweier Gewebeteile dienen müssen. Gewebe soll dabei möglichst geschont werden.

Dieses Ziel wird durch ein medizinisches Gerät nach Anspruch 1 erreicht.

### Nähere Beschreibung

Das medizinische Gerät kann durch einen Arzt oder auch durch anderes medizinisches und nicht-medizinisches Personal eingesetzt werden. Es kann zur Behandlung, insbesondere zu Operationen, bei Menschen oder auch bei Tieren eingesetzt werden. Dabei kommt die Anwendung im medizinischen oder heilenden Bereich, aber auch eine bloß kommerzielle Anwendung in Betracht. Insbesondere ist das medizinische Gerät geeignet, um Fäden in einem Gewebe zu platzieren. Beispielsweise können Fäden so platziert werden, dass sie Gewebe auffüllen. Insbesondere kommt dabei die Unterfütterung von Hautfalten in Betracht. Daher kann das medizinische Gerät vorteilhaft bei Schönheitsoperationen eingesetzt werden. Dieses medizinische Gerät ist auch geeignet, einen Faden so durch Gewebe zu führen, dass das Gewebe, insbesondere auch sein äußeres Erscheinungsbild, durch den Faden gestrafft wird. Auch hierbei kommt eine Anwendung im Rahmen von Schönheitsoperationen in Betracht.

Das medizinische Gerät soll insbesondere eine Spiralnadel umfassen, eine Nadel, die zumindest teilweise eine Spiralform hat. Die Spiralnadel weist einen spiralförmigen Nadelkörper auf, welcher um eine zentrale Achse gebildet ist. Um diese Achse herum verläuft der Nadelkörper entlang einer Spiralbahn. Der Nadelkörper weist ein vorderes Ende und ein hinteres Ende auf. Dabei ist zumindest das vordere Ende integral mit dem Nadelkörper ausgebildet. Die integrale Ausbildung kann durch (eventuell lösbare, aber im Wesentlichen) feste Verbindungen eines Teils, dass das vordere Ende ausbildet, mit dem Nadelkörper bewirkt werden. Der Übergang zwischen Nadelkörper und dem Teil, das das vordere Ende ausbildet, sollte dann glatt und im Wesentlichen fugenlos sein. Zweckmäßig wird das vordere Ende in der Regel einstückig mit dem Nadelkörper ausgebildet. Das hintere Ende kann zweckmäßigerweise ebenfalls integral mit dem Nadelkörper ausgebildet werden, wobei wiederum die einstückige Ausbildung besonders zweckmäßig ist. Das vordere Ende verläuft in Richtung der Spiralbahn des Nadelkörpers, d. h. der Verlauf des Nadelkörpers setzt sich auf das vordere Ende hin fort, ohne dass ein Knick ausgebildet wird. Das vordere Ende weist eine Spitze auf und eine dahinter angeordnete Fadenaufnahme, wobei die Spitze, oder genauer der Endpunkt oder Gipfelpunkt der Spitze, das äußere Ende des vorderen Endes markiert.

Es ist besonders zweckmäßig, wenn der spiralförmige Nadelkörper und in der Regel auch das vordere Ende und das hintere Ende massiv ausgeführt sind. Das heißt, diese Teile sind nicht aus einem Hohlkörper ausgeführt und weisen dementsprechend kein Lumen oder einen sonstigen Hohlraum auf.

Die Fadenaufnahme kann in verschiedener Form gestaltet werden, zweckmäßig ist eine Öffnung. Diese Öffnung kann rund oder oval sein. Die Öffnung kann auch schlitzförmig sein.

Entlang der Spiralbahn weist der Nadelkörper eine Außentangentenfläche und eine Innentangentenfläche auf. Die Tagentenflächen werden durch zwei Achsen festgelegt. Eine dieser Achse ist eine Tangente an die Außenseite bzw. Innenseite der Spiralbahn. Die andere Achse verläuft parallel zur zentralen Achse (A). Die Innentangentenfläche ist näher an der zentralen Achse als die Außentangentenfläche. Der spiralförmige Nadelkörper weist ferner eine Oberseite auf und eine diese gegenüberliegende Unterseite. Die Oberseite ist generell in Richtung des hinteren Endes und ggfs, eines Griffstückes orientiert.

Zweckmäßig ist es, dass die Öffnung von der Oberseite des Nadelkörpers zur Unterseite des Nadelkörpers verläuft.

Zweckmäßig ist es, wenn das vordere Ende zwischen der Außentangentenfläche und der Innentangentenfläche zugespitzt ist. Wenn man also einen Querschnitt senkrecht zu diesen Flächen betrachtet (welcher dann im Wesentlichen auch parallel zur Oberseite und zur Unterseite des Nadelkörpers verläuft), so wird in diesem Querschnitt eine Spitze sichtbar. Diese Spitze ist vorteilhafterweise dreiecksförmig. Zweckmäßig ist ein gleichschenkliges Dreieck. Zweckmäßig ist in jedem Fall, wenn der Endpunkt oder Gipfelpunkt der Spitze mittig zwischen der Außentangentenfläche und der Innentangentenfläche angeordnet ist.

Es ist ebenfalls zweckmäßig, dass das vordere Ende zwischen der Oberseite und der Unterseite des Nadelkörpers zugespitzt ist. In dieser Weise ergibt sich auch in einer Querschnittsfläche senkrecht zur Oberseite und zur Unterseite (welche also parallel zur Außentangentenfläche und zur Innentangentenfläche verläuft) eine Spitze. Dabei ist zweckmäßig, dass der Endpunkt oder der Gipfelpunkt der Spitze näher an der Oberseite liegt als an der Unterseite. Besonders zweckmäßig ist es, wenn der Gipfelpunkt ganz an der Oberseite liegt, also die Spitze in Verlängerung des Verlaufs der Spiralbahn liegt.

Es hat sich gezeigt, dass diese Art der Spitzenausbildung bei der Verwendung des medizinischen Gerätes besonders geringen Gewebeschaden verursacht und ein gut kontrollierbares und präzises Vordringen im Gewebe ermöglicht. Der Operateur erfährt dabei auch nur einen geringen Widerstand.
Es ist zweckmäßig, wenn das hintere Ende des Nadelkörpers gerade ausgebildet ist. Das hintere Ende verläuft also nicht auf einer Spiralbahn und es kann im Wesentlichen parallel zur zentralen Achse verlaufen. Besonders vorteilhaft ist es, wenn das hintere Ende auf der zentralen Achse verläuft. Dies erlaubt es, Kräfte, insbesondere Drehkräfte, besonders gut über das hintere Ende zu vermitteln. Zweckmäßig ist es, wenn das hintere Ende mit einem Griffstück verbunden ist. Über ein solches Griffstück lässt sich das medizinische Gerät gut führen und es können leicht höhere Kräfte, insbesondere Drehkräfte, aufgebracht werden.

Die Fadenaufnahme kann zweckmäßigerweise die Form eines Loches haben und zwar im Wesentlichen die Form eines runden oder eines ovalen Loches. Besonders vorteilhaft ist es, wenn eine Fadenaufnahme ein Loch, zwei Löcher oder drei Löcher umfasst.

Alternativ oder zusätzlich kann die Fadenaufnahme auch als Schlitz ausgebildet sein. Zweckmäßigerweise ist ein im Wesentlichen gerader Schlitz, also ein Schlitz mit rechteckigem Querschnitt. Wenn eine längliche Rechteckform gebildet wird, kann es auch günstig sein, an zumindest einem kurzen Enden eines solchen Rechteckes eine Ausbuchtung vorzusehen, beispielsweise eine kreisförmige Ausbuchtung. Solche Ausbuchtungen können auch an beiden gegenüberliegenden kurzen Enden ausgebildet werden. Ein zweckmäßiger Schlitz kann auch eine im Querschnitt ovale Form haben.

Es kann zweckmäßig sein, wenn das vordere Ende des medizinischen Gerätes einen bauchigen Abschnitt aufweist. Ein solcher bauchiger Abschnitt kann insbesondere in Höhe der Fadenaufnahme vorgesehen werden. Entlang des bauchigen Abschnittes erhöht sich der Durchmesser des Nadelkörpers (entlang einer Kreisscheibe gemessen, welche senkrecht zur Spiralbahn steht).
Nützlich ist auch ein medizinisches Gerät, welches bereits einen chirurgischen Faden aufweist. Dieser Faden ist zweckmäßigerweise bereits durch die Fadenaufnahme geführt. Es ist besonders nützlich, wenn ein solches Gerät zusammen mit dem Faden in steriler Verpackung ausgeliefert wird. Dabei kann das Gerät einen Faden aufweisen oder auch mehr als einen Faden, beispielsweise zwei, drei oder vier Fäden.

Im Rahmen der vorliegenden Erfindung ist auch ein Satz medizinischer Geräte nützlich, welcher zwei, drei oder mehr Geräte umfasst. Diese Geräte können jeweils mit oder ohne Fäden zur Verfügung gestellt werden. Die Geräte können gleichartig oder auch verschiedenartig ausgeführt sein. Es ist beispielsweise nützlich, ein medizinisches Gerät mit einer links-drehenden Spiralnadel und ein medizinisches Gerät mit einer rechts-drehenden Spiralnadel zu kombinieren. In dieser Weise kann ein Satz mit zwei sich ergänzenden medizinischen Geräten zur Verfügung gestellt werden.

Es ist auch zweckmäßig, medizinische Geräte mit verschiedenem Durchmesser des Nadelkörpers zur Verfügung zu stellen. Ebenfalls kann die Länge des Gerätes entlang der zentralen Achse A variiert werden. Ferner kann der Durchmesser des Nadelkörpers um die zentrale Achse herum variiert werden. Auch können medizinische Geräte aus verschiedenen Materialien, beispielsweise mit verschiedener Materialhärte, eingesetzt werden. Dabei wird in der Regel chirurgischer Stahl verwendet, es sind aber auch andere Materialien zur Herstellung des medizinischen Gerätes denkbar, beispielsweise Kunststoffe. Alle diese Materialien sind in verschiedener Materialhärte verfügbar.

Es kann auch ein Gerät zweckmäßig sein, bei dem sich der Spiralabstand entlang der zentralen Achse ändert. Das bedeutet, dass ein 360-Grad-Umlauf der Spirale um die zentrale Achse über variable Längenabschnitte (gemessen auf der zentralen Achse) stattfindet.

Geräte mit diesen Variationen sind jeweils einzeln für bestimmte Anwendungsgebiete nützlich, sie lassen sich aber auch sinnvoll in einem Gerätesatz kombinieren.

Weitere Merkmale, aber auch Vorteile der Erfindung, ergeben sich aus den nachfolgend aufgeführten Zeichnungen und der zugehörigen Beschreibung. In den Abbildungen und in den dazugehörigen Beschreibungen sind Merkmale der Erfindung in Kombination beschrieben. Diese Merkmale können allerdings auch in anderen Kombinationen von einem erfindungsgemäßen Gegenstand umfasst werden. Jedes offenbarte Merkmal ist also auch als in technisch sinnvollen Kombinationen mit anderen Merkmalen offenbart zu betrachten. Die Abbildungen sind teilweise leicht vereinfacht und schematisch.
- Fig. 1: zeigt eine perspektivische Gesamtansicht eines erfindungsgemäßen medizinischen Gerätes.
- Fig. 2: zeigt eine perspektivische Ansicht des Nadelkörpers des Gerätes.
- Fig. 3: zeigt in zwei Querschnittsansichten das vordere Ende des Nadelkörpers eines erfindungsgemäßen Gerätes.
- Fig. 4: zeigt in zwei Querschnittsansichten das vordere Ende eines anderen erfindungsgemäßen medizinischen Gerätes.
- Fig. 5: zeigt in zwei Querschnittsansichten das vordere Ende eines noch anderen erfindungsgemäßen medizinischen Gerätes.
- Fig. 6: zeigt in einer Querschnittsansicht das vordere Ende eines wiederum anderen medizinischen Gerätes.
- Fig. 7: zeigt in entsprechender Querschnittsansicht das vordere Ende eines noch anderen medizinischen Gerätes.
- Fig. 8: zeigt in entsprechender Querschnittsansicht das vordere Ende eines wiederum anderen medizinischen Gerätes.
- Fig. 9: zeigt in perspektivischer Ansicht den Nadelkörper eines erfindungsgemäßen Gerätes zusammen mit einem chirurgischen Faden.

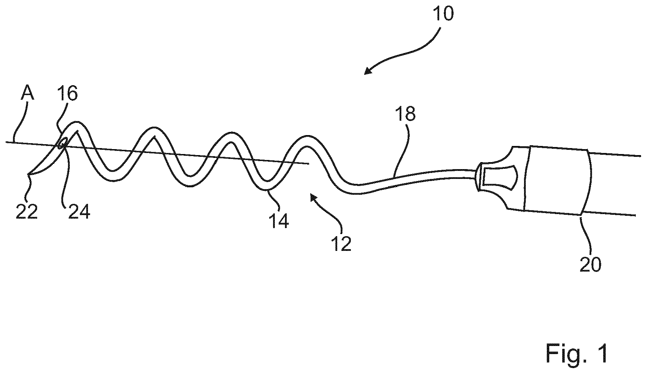

Fig. 1 zeigt in perspektivischer Ansicht ein medizinisches Gerät 10 nach der vorliegenden Erfindung. Das Gerät weist eine Spiralnadel 12 auf. Diese Spiralnadel umfasst einen spiralförmigen Nadelkörper 14, welcher von einem vorderen Ende 16 zu einem hinteren Ende 18 verläuft. Der spiralförmige Nadelkörper verläuft entlang einer Spirale, welche sich um eine zentrale Achse A erstreckt. Das hintere Ende 18 ist mit einem Griffstück 20 verbunden. Am vorderen Ende 16 weist das medizinische Gerät 10 eine Spitze 22 auf. Ebenfalls im Bereich des vorderen Endes, aber hinter der Spitze 22, ist eine Fadenaufnahme 24 angeordnet.

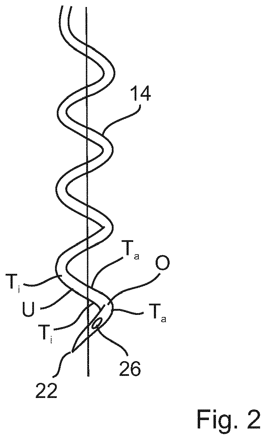

Fig. 2 zeigt in einer vergrößerten perspektivischen Ansicht den Verlauf des spiralförmigen Nadelkörpers 14. Die Spirale windet sich entlang einer Spiralbahn vom hinteren Ende (nicht dargestellt) in Richtung auf die Spitze 22. Hinter der Spitze 22 ist die Fadenaufnahme vorgesehen, welche hier die Form eines einzelnen Loches 26 hat. Entlang der Spiralbahn lassen sich gedanklich beliebig viele Scheiben als Querschnitte durch den Nadelkörper denken. Für jede dieser Scheiben lässt sich eine Außentangentenfläche und eine Innentangentenfläche sowie eine Oberseite und eine Unterseite definieren. Dabei wird die Außentangentenfläche durch eine äußere Tangente an die Scheibe (und senkrecht zur Scheibenfläche) und durch die zentrale Achse definiert. Die innere Tangentenfläche wird in entsprechender Weise durch die innere Tangente an die Scheibe (genauer: an einen in der Scheibe liegenden Ring, dessen radiale Dicke durch den Nadelkörper bestimmt wird) und die zentrale Achse definiert. Um ca. 90 Grad versetzte Bereiche des Nadelkörpers lassen sich als Oberseite bzw. als Unterseite der Nadel beschreiben. Durch die entsprechenden Markierungen in Fig. 2, Tₐ für die äußere Tangentenfläche, Tᵢ für die innere Tangentenfläche, O für Oberseite, U für Unterseite, sind die entsprechenden Flächen leicht erkennbar (wenngleich die Aufsicht auf die Unterseite U in dieser Darstellung nicht möglich ist). In analoger Weise sind sie für eine Nadel etwas anderer Form festzulegen.

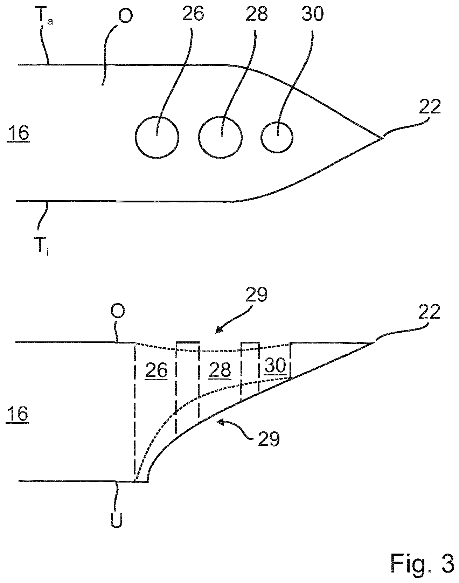

Fig. 3 zeigt in zwei Querschnittsansichten das vordere Ende 16 eines erfindungsgemäßen Nadelkörpers. Das vordere Ende 16 wird durch eine Spitze 22 begrenzt. Zur Orientierung sind eine Außentangentenfläche, eine Innentangentenfläche und die Oberseite sowie (in der anderen Querschnittsansicht) auch die Unterseite eingezeichnet. Die Fadenaufnahme ist in Form von drei Löchern vorgesehen, nämlich eines ersten Loches 26, eines zweiten Loches 28 und eines dritten Loches 30.

Wie in der zweiten Querschnittsansicht gut sichtbar, verbinden diese Löcher die Oberseite des Nadelkörpers mit der Unterseite des Nadelkörpers. Allgemein ist es im Rahmen der vorliegenden Erfindung zweckmäßig, wenn das vordere Ende ungefähr im Bereich der Fadenaufnahme eine Einbuchtung 29 aufweist. Diese erleichtert die Führung der Spitze und schont Gewebe. Die Randbereiche der entsprechenden Einbuchtung sind in der Querschnittsansicht durch gestrichelte Linien markiert. Entlang dieser Linien können Sicken verlaufen, welche die Einbuchtung seitlich begrenzen. Wenngleich dieses zusätzliche Merkmal nur im Zusammenhang mit Fig. 3 gezeigt wird, so ist es ohne weiteres auch bei den anderen dargestellten Ausführungsformen nützlich.

In den beiden Querschnittsansichten ist auch die Spitzenform gut erkennbar. Zwischen der Außentangentenfläche und der Innentangentenfläche spitzt sich das vordere Ende im Gipfelpunkt zu. Dieser liegt mittig zwischen der Außentangentenfläche und der Innentangentenfläche. Das vordere Ende 16 spitzt sich ebenfalls zwischen Oberseite O und Unterseite U zu. Diese Spitze liegt allerdings nicht mittig zwischen diesen beiden Seiten, sondern vielmehr ganz auf der Seite der Oberseite.

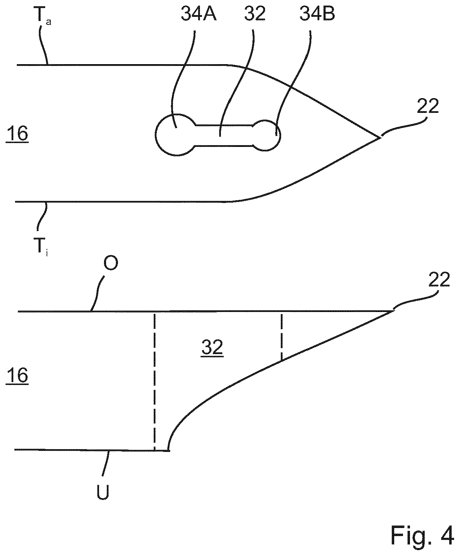

Fig. 4 zeigt in zwei Querschnittsansichten, welcher denen der Fig. 3 entsprechen, eine andere Ausführungsform des erfindungsgemäßen medizinischen Gerätes. Dies weist eine abweichende Gestaltung des vorderen Endes 16 auf. Die Form der Spitze 22 ist allerdings ebenso gewählt wie bei dem Gerät aus Fig. 3. Statt einer Vielzahl von Löchern ist bei diesem Gerät jedoch ein Schlitz 32 vorgesehen. Dieser Schlitz weist im Wesentlichen, wie in der oberen Querschnittsansicht gut sichtbar, im Querschnitt die Form eines länglichen Rechtecks auf. An den schmalen Enden des Rechtecks sind jedoch lochartige Ausbuchten 34A und 34B vorgesehen. Diese erleichtern die Aufnahme eines Fadens.

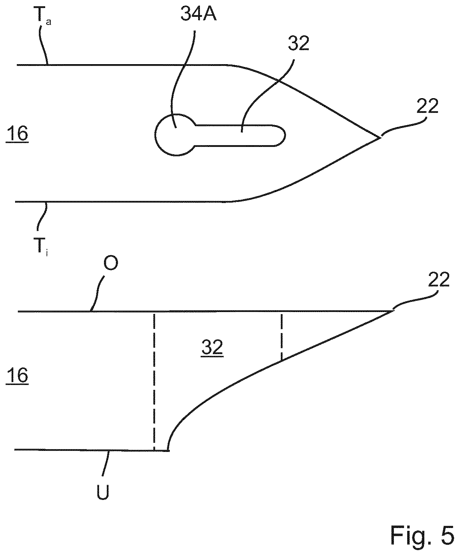

Fig. 5 zeigt in zwei Querschnittsansichten, welche denen der Fig. 3 entsprechen, eine wiederum andere Ausführungsform eines erfindungsgemäßen medizinischen Gerätes. Bei diesem Gerät ist wiederum ein Schlitz 32 vorgesehen. Der Schlitz hat ebenfalls im Querschnitt die Form eines länglichen Rechtecks. Er weist jedoch nur an einem schmalen Ende des Rechtecks eine lochartige Ausbuchtung 34A auf. Diese Ausbuchtung erleichtert die Aufnahme eines Fadens, der dann am engeren Ende des Schlitzes sehr sicher geführt wird.

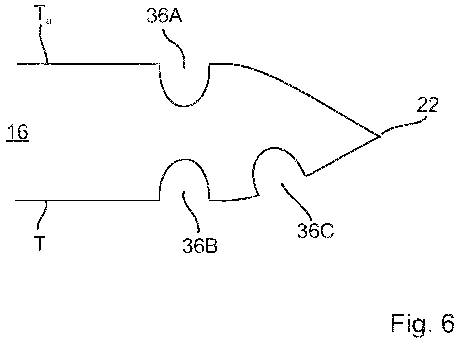

Fig. 6 zeigt das vordere Ende 16 eines anderen erfindungsgemäßen medizinischen Gerätes. Dieses weist keine Löcher auf, sondern vielmehr mehrere Nuten, nämlich Nut 36A, gegenüberliegend Nut 36B, und noch näher zur Spitze 22 hin eine dritte Nut 36C. Auch an diesen Nuten kann ein chirurgischer Faden fest verankert werden.

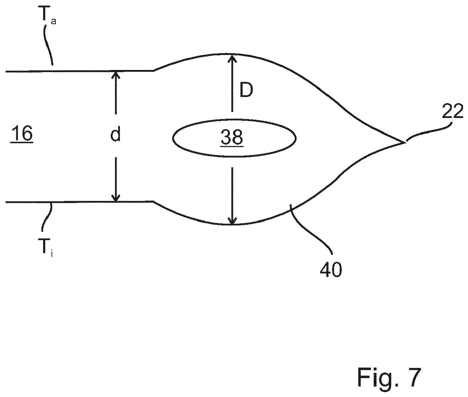

Fig. 7 zeigt eine andere Ausführungsform eines erfindungsgemäßen medizinischen Gerätes. In diesem Fall ist für die Fadenaufnahme ein ovaler Schlitz 38 vorgesehen. Ferner weist das vordere Ende 16 des Nadelkörpers einen bauchigen Bereich auf. In diesem Bereich ist der Durchmesser des Nadelkörpers gemessen senkrecht zur Spiralbahn verbreitert.

Dementsprechend ergibt sich vor dem bauchigen Bereich 14 der Durchmesser d, während sich im bauchigen Bereich der größere Durchmesser D ergibt.

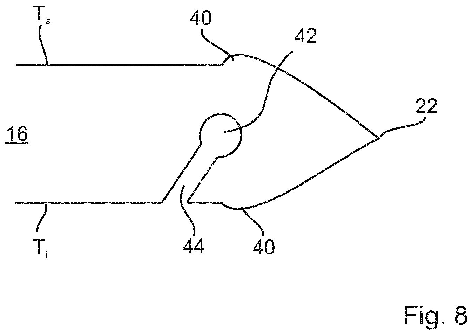

Fig. 8 zeigt in einer Querschnittsansicht eine noch andere Ausführungsform eines erfindungsgemäßen medizinischen Gerätes. In diesem Fall ist am vorderen Ende hinter der Spitze 22 ein Loch 42 aufgenommen. In dieses muss der Faden jedoch nicht eingefädelt werden, da das Loch durch die Kerbe 44 mit der Innentangentialfläche des Nadelkörpers verbunden ist. Wiederum ist in der Nachbarschaft des Loches ein bauchiger Bereich 40 vorgesehen.

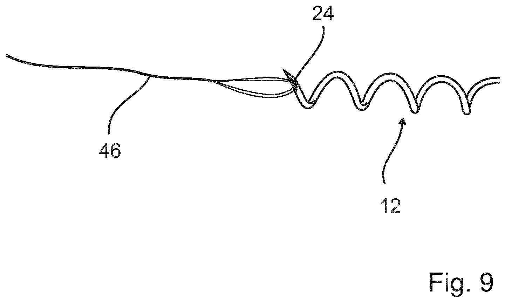

Fig. 9 zeigt die Spiralnadel eines erfindungsgemäßen medizinischen Gerätes, welche in ihrer Fadenaufnahme 24 einen chirurgischen Faden 46 trägt. Es ist zweckmäßig, dass ein etwa mittiger Abschnitt des chirurgischen Fadens 46 von der Fadenaufnahme 24 aufgenommen wird. Beide Enden des chirurgischen Fadens können dann nebeneinander geführt oder sogar verzwirbelt werden. In dieser Weise hat der chirurgische Faden 46 einen sicheren Sitz in der Fadenaufnahme 24. Ferner ist es möglich, so die doppelte Fadenstärke, nämlich gleichzeitig zwei Enden des Fadens, im Gewebe einzuführen.

Insgesamt erkennt man, wie durch die vorliegende Erfindung ein zweckmäßiges medizinisches Gerät sich zur Verfügung stellen lässt, welches für die eingangs beschriebenen Eingriffe besonders geeignet ist.

### Bezugszeichenliste

- 10: Medizinisches Gerät
- 12: Spiralnadel
- 14: Nadelkörper
- 16: Vorderes Ende
- 18: Hinteres Ende
- 20: Griffstück
- 22: Spitze
- 24: Fadenaufnahme
- 26: Loch
- 28: Loch
- 29: Vertiefung
- 30: Loch
- 32: Schlitz
- 34: (lochförmige) Ausbuchtung
- 36: Nut
- 38: Ovalschlitz
- 40: Bauch
- 42: Loch
- 44: Kerbe/Durchführung
- 46: Chirurgischer Faden
- A: Zentrale Achse
- Tₐ: Außentangentenfläche
- Tᵢ: Innentangentenfläche
- O: Oberseite
- U: Unterseite

## Patentansprüche

1. Medizinisches Gerät (10), welches eine Spiralnadel (12) umfasst, wobei die Spiralnadel (12) einen spiralförmigen Nadelkörper (14)
aufweist, welcher um eine zentrale Achse (A) gebildet ist und entlang
einer Spiralbahn verläuft und ferner ein vorderes Ende (16) und ein hinteres Ende (18) aufweist, wobei zumindest das vordere Ende (16) integral mit dem Nadelkörper (14) ausgebildet ist, wobei die Spiralbahn eine Außentangentenfläche (Ta), eine
Innentangentenfläche (Ti), eine Oberseite (O) und eine dieser gegenüberliegende Unterseite (U) aufweist, und wobei das vordere Ende (16) in Richtung der Spiralbahn des Nadelkörpers (14) verläuft, und das vordere Ende (16) eine Spitze (22) und eine dahinter angeordnete Fadenaufnahme (24) aufweist, wobei die Fadenaufnahme (24) eine Öffnung (26, 28, 30, 32, 38, 42) aufweist und wobei das vordere Ende (16) zwischen der Oberseite (O) und der Unterseite (U) zugespitzt ist und zwischen Oberseite (O) und der Unterseite (U) ein Gipfelpunkt ausgebildet ist, **dadurch gekennzeichnet, dass** die Öffnung (26, 28, 30, 32, 38, 42) von der Oberseite (O) zur Unterseite (U) verläuft, und dass der Gipfelpunkt näher an der Oberseite (O) als an der Unterseite (U) liegt.

2. Medizinisches Gerät (10) nach dem vorhergehenden Anspruch, wobei der Nadelkörper (14) massiv ausgebildet ist.

3. Medizinisches Gerät (10) nach einem der vorhergehenden Ansprüche, wobei das vordere Ende (16) zwischen der Außentangentenfläche (Ta) und der Innentangentenfläche (Ti) zugespitzt ist.

4. Medizinisches Gerät (10) nach dem vorhergehenden Anspruch, wobei der Gipfelpunkt mittig zwischen der Außentangentenfläche (Ta) und der Innentangentenfläche (Ti) ausgebildet wird.

5. Medizinisches Gerät (10) nach einem der vorhergehenden Ansprüche, bei dem das hintere Ende (18) gerade verläuft.

6. Medizinisches Gerät (10) nach dem vorhergehenden Anspruch, wobei das hintere Ende (18) zumindest abschnittsweise auf der zentralen Achse (A) verläuft.

7. Medizinisches Gerät (10) nach einem der vorhergehenden Ansprüche, bei dem das hintere Ende (18) mit einem Griffstück (20) verbunden ist.

8. Medizinisches Gerät (10) nach einem der vorhergehenden Ansprüche, bei dem die Fadenaufnahme (24) zwischen ein und drei Löcher umfasst.

9. Medizinisches Gerät (10) nach einem der vorhergehenden Ansprüche, bei dem die Fadenaufnahme (24) einen Schlitz (32, 38) umfasst.

10. Medizinisches Gerät (10) nach einem der vorhergehenden Ansprüche, bei dem das vordere Ende (16) im Bereich der Fadenaufnahme (24) bauchig verbreitert ist.

11. Medizinisches Gerät (10) nach einem der vorhergehenden Ansprüche, bei dem am vorderen Ende (16) im Bereich der Fadenaufnahme (24) eine Einbuchtung (29) vorgesehen ist.

12. Medizinisches Gerät (10) nach einem der vorhergehenden Ansprüche, bei dem die Fadenaufnahme (24) einen chirurgischen Faden (44) führt.

## Claims

1. Medical device (10) which comprises a spiral needle (12), the spiral needle (12) having a spiral-shaped needle body (14) which is formed about a central axis (A), extends along a spiral path and also has a front end (16) and a rear end (18), at least the front end (16) being integrally formed with the needle body (14), the spiral path having an outer tangent surface (Ta), an inner tangent surface (Ti), an upper side (O) and an underside (U) opposite thereto, and the front end (16) extending in the direction of the spiral path of the needle body (14), and the front end (16) having a tip (22) and a thread receptacle (24) arranged therebehind, the thread receptacle (24) having an opening (26, 28, 30, 32, 38, 42) and the front end (16) being tapered between the upper side (O) and the underside (U) and a peak point being formed between the upper side (O) and the underside (U), **characterised in that** the opening (26, 28, 30, 32, 38, 42) extends from the upper side (O) to the underside (U), and **in that** the peak point is closer to the upper side (O) than to the underside (U).

2. Medical device (10) according to the preceding claim, wherein the needle body (14) is solid.

3. Medical device (10) according to either of the preceding claims, wherein the front end (16) is tapered between the outer tangent surface (Ta) and the inner tangent surface (Ti).

4. Medical device (10) according to the preceding claim, wherein the peak point is formed centrally between the outer tangent surface (Ta) and the inner tangent surface (Ti).

5. Medical device (10) according to any of the preceding claims, wherein the rear end (18) extends in a straight line.

6. Medical device (10) according to the preceding claim, wherein the rear end (18) extends at least in portions over the central axis (A).

7. Medical device (10) according to any of the preceding claims, wherein the rear end (18) is connected to a handle (20).

8. Medical device (10) according to any of the preceding claims, wherein the thread receptacle (24) comprises between one and three holes.

9. Medical device (10) according to any of the preceding claims, wherein the thread receptacle (24) comprises a slot (32, 38).

10. Medical device (10) according to any of the preceding claims, wherein the front end (16) is widened in a bulbous manner in the region of the thread receptacle (24).

11. Medical device (10) according to any of the preceding claims, wherein a recess (29) is provided at the front end (16) in the region of the thread receptacle (24).

12. Medical device (10) according to any of the preceding claims, wherein the thread receptacle (24) guides a surgical thread (44).

## Revendications

1. Appareil médical (10), qui comprend une aiguille spiralée (12), l'aiguille spiralée (12) comportant un corps d'aiguille (14) en forme de spirale qui est formé autour d'un axe central (A) et s'étend le long d'une trajectoire spiralée et qui comporte en outre une extrémité avant (16) et une extrémité arrière (18), au moins l'extrémité avant (16) étant constituée d'un seul tenant avec le corps d'aiguille (14), la trajectoire spiralée comportant une
surface tangente extérieure (Ta), une
surface tangente intérieure (Ti), un côté supérieur (O) et un côté inférieur (U) opposé à celui-ci, et l'extrémité avant (16) s'étendant en direction de la trajectoire spiralée du corps d'aiguille (14), et l'extrémité avant (16) comportant une pointe (22) et un logement pour fil (24) disposé derrière, le logement pour fil (24) comportant une ouverture (26, 28, 30, 32, 38, 42), et
l'extrémité avant (16) étant effilée entre le côté supérieur (O) et le côté inférieur (U), et un point culminant étant constitué entre le côté supérieur (O) et le côté inférieur (U), **caractérisé en ce que** l'ouverture (26, 28, 30, 32, 38, 42) s'étend du côté supérieur (O) vers le côté inférieur (U), et **en ce que** le point culminant est situé plus près du côté supérieur (O) que du côté inférieur (U).

2. Appareil médical (10) selon la revendication précédente, le corps d'aiguille (14) étant constitué de façon massive.

3. Appareil médical (10) selon l'une des revendications précédentes, l'extrémité avant (16) étant effilée entre la surface tangente extérieure (Ta) et la surface tangente intérieure (Ti).

4. Appareil médical (10) selon la revendication précédente, le point culminant étant constitué de façon centrée entre la surface tangente extérieure (Ta) et la surface tangente intérieure (Ti).

5. Appareil médical (10) selon l'une des revendications précédentes, dans lequel l'extrémité arrière (18) s'étend de façon rectiligne.

6. Appareil médical (10) selon la revendication précédente, l'extrémité arrière (18) s'étendant au moins par tronçons sur l'axe central (A).

7. Appareil médical (10) selon l'une des revendications précédentes, dans lequel l'extrémité arrière (18) est raccordée à une poignée (20).

8. Appareil médical (10) selon l'une des revendications précédentes, dans lequel le logement pour fil (24) comprend entre un et trois trous.

9. Appareil médical (10) selon l'une des revendications précédentes, dans lequel le logement pour fil (24) comprend une fente (32, 38).

10. Appareil médical (10) selon l'une des revendications précédentes, dans lequel l'extrémité avant (16) s'élargit de façon bombée dans la zone du logement pour fil (24).

11. Appareil médical (10) selon l'une des revendications précédentes, une échancrure (29) étant prévue sur l'extrémité avant (16) dans la zone du logement pour fil (24).

12. Appareil médical (10) selon l'une des revendications précédentes, dans lequel le logement pour fil (24) guide un fil chirurgical (44).
